# FASCICULE DE BREVET EUROPEEN

(11) **EP 2 384 737 B1**
(45) Date de publication et mention de la délivrance du brevet: **02.10.2013**
(21) Numéro de dépôt: 10172540.6
(22) Date de dépôt: 11.08.2010
(51) Int. Cl.: A61K 8/31, A61K 8/89, A61Q 5/06, A61Q 5/12, A61K 8/891

(54) **Composition cosmétique comprenant un copolymère siliconé particulier et au moins deux alcanes linéaires volatils liquides**
KOSMETISCHE ZUSAMMENSETZUNG ENTHALTEND EIN SPEZIFISCHES SILIKONCOPOLYMER UND MINDESTENS ZWEI FLÜSSIGE GERADKETTIGE FLÜCHTIGE ALKANE
COSMETIC COMPOSITION COMPRISING A SPECIFIC SILICONE COPOLYMER AND AT LEAST TWO LIQUID LINEAR VOLATILE ALKANES

(30) Priorité: 13.08.2009 FR 0955660
(43) Date de publication de la demande: 09.11.2011
(73) Titulaire: L'Oréal, 75008 Paris (FR)
(72) Inventeur: Bonnamy, Arnaud, 78000, Versailles (FR); Brun, Gaëlle, 75011, Paris (FR)
(74) Mandataire: Fevrier, Murielle Françoise E.

(56) Documents cités:
- EP-A1- 2 016 933
- EP-A2- 0 903 388
- WO-A2-2004/073626
- DE-A1-102008 012 457
- US-B1- 6 280 851

## Description

La présente invention a pour objet une composition de traitement des fibres kératiniques, et en particulier des fibres kératiniques humaines telles que les cheveux, ainsi qu'un procédé de traitement des fibres kératiniques mettant en oeuvre cette composition.

Il existe de nombreux produits de coiffage permettant d'apporter du corps, de la masse ou du volume aux cheveux. Un inconvénient lié à ces produits, le plus souvent à base de polymères filmogènes, réside dans le fait que l'effet cosmétique disparaît dès le premier shampooing.

Il est connu de réaliser des gainages sur cheveux avec des polymères filmogènes tels que des copolymères de silicone particuliers à base de résine de silicone et de silicone fluide, plus connus sous le nom de BioPSA. Ces copolymères sont notamment décrits dans les demandes de brevet WO 03/026596, WO 2004/073626, WO 2007/051505 et WO 2007/051506 pour diverses applications cosmétiques telles que l'application sur les cheveux, les ongles, la peau.

Il est possible d'apporter un coiffage rémanent en utilisant ce type de polymère. Lorsque ces polymères sont appliqués sur les cheveux dans un solvant volatil, les cheveux sont gainés de manière homogène tout en restant individualisés, le gainage obtenu étant rémanent aux shampooings. Ce gainage apporte à la chevelure du corps, de la masse de manière rémanente.

Lorsque ces polymères sont utilisés en mélange avec des pigments, on obtient généralement une coloration visible quelque soit la couleur initiale des cheveux. Et ceci sans décoloration préalable des cheveux. La présence de ces pigments, notamment des nacres permet d'obtenir une coloration chromatique et visible sur cheveux. Le gainage obtenu est rémanent à quelques shampooings.

Cependant pour que la cosmétique et l'homogénéité du gainage obtenu soient bonnes, la vitesse d'évaporation de la composition est une étape clé. La vitesse d'évaporation ne doit pas être trop rapide sinon la composition ne s'étale pas bien sur le long de la mèche et le gainage ne se forme pas de manière homogène, ce qui se traduit notamment par un brushing plus difficile à réaliser. La vitesse d'évaporation ne doit pas être trop lente, sinon il restera encore du solvant piégé dans le gainage après l'étape de séchage ce qui va dégrader la cosmétique du gainage. Les huiles volatiles, comme l'isododécane, utilisées couramment avec les copolymères à base de résine de silicone et de silicone fluide, ont une vitesse d'évaporation trop élevée.

Ainsi, le but de la présente invention est de développer des gainages homogènes à partir de compositions comprenant des solvants présentant une vitesse d'évaporation appropriée pour le gainage capillaire.

Ce but est atteint avec la présente invention qui a pour objet une composition de traitement des fibres kératiniques comprenant un ou plusieurs copolymères à base de résine de silicone et de silicone fluide, au moins deux alcanes linéaires volatils liquides distincts, différant entre eux d'un nombre de carbone n d'au moins 1, les alcanes linéaires volatils liquides comprenant de 9 à 15 atomes de carbone, le rapport en poids entre les alcanes linéaires volatils liquides et le copolymère à base de résine de silicone et de silicone fluide étant supérieur ou égal à 2. L'invention a aussi pour objet un procédé de traitement des fibres kératiniques à partir de cette composition.

L'utilisation d'alcanes linéaires volatils liquides associée au copolymère siliconé décrit ci-dessus permet d'optimiser la vitesse d'évaporation facilitant ainsi l'application de la composition et garantissant une bonne homogénéité du gainage sur les cheveux.

Ce procédé lorsqu'il est mis en oeuvre avec une composition comprenant un ou plusieurs pigments permet d'obtenir des gainages homogènes ayant une coloration intense et/ou chromatique, notamment sur des fibres kératiniques foncées.

Dans le cadre de l'invention, les fibres kératiniques ou cheveux foncés présentent une hauteur de ton inférieure ou égale à 6 (blond foncé) et de préférence inférieure ou égale à 4 (châtain).

La notion de « hauteur de ton » repose sur la classification des nuances naturelles, un ton séparant chaque nuance de celle qui la suit ou la précède immédiatement. Cette définition et la classification des nuances naturelles est bien connue des professionnels de la coiffure et publiée dans l'ouvrage « Sciences des traitements capillaires » de Charles ZVIAK 1988, Ed.Masson, pp.215 et 278.

Les hauteurs de ton s'échelonnent de 1 (noir) à 10 ( blond clair clair), une unité correspondant à un ton ; plus le chiffre est élevé et plus la nuance est claire.

Dans ce qui suit, sauf indication contraire, les bornes des intervalles indiqués sont comprises dans l'invention.

### Copolymère à base de résine de silicone et de silicone fluide

Le copolymère siliconé défini selon l'invention est dérivé de la réaction entre une résine de silicone et une silicone fluide.

De tels copolymères sont décrits par exemple dans « Silicone Pressure Sensitive Adhesive », Sobieski and Tangney, Handbook of Pressure Sensitive Adhesive Technology (D. Satas Ed.), Von Nostrand Reinhold, New York.

Dans le copolymère, la résine de silicone est présente à un taux compris entre 45 et 75 % (par rapport à la masse totale de silicone) et la silicone fluide est présente à un taux compris entre 25 et 55 %, avec la somme des pourcentages de résine de silicone et de silicone fluide qui est égal à 100. De préférence, la résine de silicone est présente à un taux compris entre 55 et 65 % (par rapport à la masse totale de silicone) et la silicone fluide est présente à un taux compris entre 35 et 45 %, avec la somme des pourcentages de résine de silicone et de silicone fluide qui est égal à 100.

De préférence, la résine de silicone selon l'invention est le produit de condensation de groupements SiO₂ et de groupements R₃(SiO)_{1/2} (triorganosilyle) pour lequel chaque groupement R est indépendamment sélectionné parmi les radicaux méthyle, éthyle, propyle ou vinyle et pour lequel le rapport entre les fonctions SiO₂ et les fonctions R₃(SiO)_{1/2} de la résine de silicone va de 0,6 à 0,9. Des groupements triorganosilyle utilisables pour former la résine de silicone peuvent être des unités triméthylsilyle, triéthylsilyle, méthylméthylproprylsilyle, diméthylvinylsilyle et des mélanges de celles-ci. Le groupement triméthylsilyle est le préféré dans le cadre de l'invention.

De préférence, la silicone fluide selon l'invention est une diorganopolysiloxane aux fonctions OH terminales ayant une viscosité comprise entre 100 et 100 000 cst à 25 °C pour laquelle les substituants de la diorganopolysiloxane sont indépendamment choisis parmi les radicaux méthyle, éthyle, propyle ou vinyle. Les diorganopolysiloxanes sont de préférence des polymères linéaires. Des exemples de diorganopolysiloxane peuvent être, de manière non limitative, une polydiméthylsiloxane, une éthylméthylpolysiloxane, un copolymère de diméthylsiloxane et de méthylvinylsiloxane, et des mélanges de tels polymères ou copolymères ayant des extrémités OH. La diorganopolysiloxane préférée est une polydiméthylsiloxane.

Des exemples de synthèse d'un tel copolymère sont par exemple décrits dans le brevet US 5 162 410 ou dans le brevet CA 711756.

Les copolymères selon la présente invention peuvent donc être préparés en chauffant le mélange suivant :
- de 45 % à 75 % en masse de résine de silicone étant le produit de condensation des unités SiO₂ et R₃(SiO)_{1/2} pour lequel chaque groupement R est indépendamment sélectionné parmi les radicaux méthyle, éthyle, propyle ou vinyle et pour lequel le rapport entre les fonctions SiO₂ et les fonctions R₃(SiO)_{1/2} de la résine de silicone va de 0,6 à 0,9 ;
- de 25 % à 55 % en masse de diorganopolysiloxane fluide aux fonctions OH terminales ayant une viscosité comprise entre 100 et 100 000 cst à 25 °C pour laquelle les substituants de la diorganopolysiloxane sont indépendamment choisis parmi les radicaux méthyle, éthyle, propyle ou vinyle ;
- de 0,001 % à 5 % d'un catalyseur adapté, qui est de préférence un composé aminé aliphatique organique choisi de préférence parmi les amines primaires, les amines secondaires, les amines tertiaires, les sels d'acide carboxylique des amines citées ci-dessus et les sels d'ammonium quaternaires.

Le mélange est chauffé à une température comprise entre 80 °C et 160 °C jusqu'à ce que le caractère adhésif du copolymère siliconé résultant soit obtenu.

Les copolymères préférés selon l'invention sont commercialisés par Dow Corning sous la référence BIO-PSA^{®}, ces BIO-PSA^{®} pouvant être sous deux formes, standard ou amine compatible, et étant fournis dans différents solvants avec plusieurs ratio résine de silicone / silicone fluide. On peut notamment citer les grades 7-4400, 7-4500, 7-4600. Le BIO-PSA^{®} particulièrement préféré selon l'invention est le grade 7-4400.

Selon un mode de réalisation particulier de l'invention, la quantité de copolymère est supérieure à 1 % en poids du poids total de la composition.

Le copolymère peut notamment être présent dans la composition selon l'invention en une teneur supérieure à 1 % et jusqu'à 80 % en poids par rapport au poids total de la composition, de préférence allant de 1,5 % à 40 % en poids, et préférentiellement allant de 1,5 % à 20 % en poids.

### Alcanes linéaires volatils

Selon un mode de réalisation, un alcane linéaire volatil convenant à l'invention peut présenter un point éclair compris dans l'intervalle variant de 30 à 120 °C, et plus particulièrement de 40 à 100 °C,.

Un alcane linéaire volatil convenant à l'invention est liquide à température ambiante (environ 25 °C) et à la pression atmosphérique (760 mmHg).

Un alcane convenant à l'invention est un alcane linéaire volatil comprenant de 9 à 15 atomes de carbone, en particulier de 10 à 15 atomes de carbone, et plus particulièrement de 11 à 13 atomes de carbone.

Un alcane linéaire volatil convenant à l'invention peut être avantageusement d'origine végétale.

Un tel alcane peut être obtenu, directement ou en plusieurs étapes, à partir d'une matière première végétale comme une huile, un beurre, une cire, etc.

A titre d'exemple d'alcane convenant à l'invention, on peut mentionner les alcanes décrits dans les demandes de brevets de la société Cognis WO 2007/068371, ou WO2008/155059 (mélanges d'alcanes distincts et différant d'au moins un carbone). Ces alcanes sont obtenus à partir d'alcools gras, eux-mêmes obtenus à partir d'huile de coprah ou de palme.

A titre d'exemple d'alcane linéaire convenant à l'invention, on peut citer le n-nonane (C9), le n-décane (C10), le n-undécane (C11), le n-dodécane (C12), le n-tridécane (C13), le n-tétradecane (C14), le n-pentadécane (C15), et leurs mélanges, et en particulier le mélange de n-undécane (C11) et de n-tridécane (C13) décrit à l'exemple 1 de la demande WO2008/155059 de la Société Cognis, et le mélange n-dodécane (C12) et de n-tétradécane (C14) vendu par SASOL, sous les références PARAFOL 12-97 et PARAFOL 14-97, respectivement dodécane et tétradécane linéaires, ainsi que leurs mélanges.

On utilisera l'alcane linéaire volatil en mélange d'au moins deux alcanes volatils distincts et différant entre eux d'un nombre de carbone d'au moins 1.

Selon un premier mode de réalisation, on utilise un mélange d'au moins deux alcanes linéaires volatils distincts comportant de 10 à 15 atomes de carbone et différant entre eux d'un nombre de carbone d'au moins 1. A titre d'exemples, on peut citer notamment les mélanges d'alcanes linéaires volatils C10/C11, C11/C12, C12/C13, ou C14/C15.

Selon un autre mode de réalisation, on utilise un mélange d'au moins deux alcanes linéaires volatils distincts comportant de 10 à 15 atomes de carbone et différant entre eux d'un nombre de carbone d'au moins 2. A titre d'exemples, on peut citer notamment les mélanges d'alcanes linéaires volatils C10/C12, ou C12/C14, pour un nombre de carbone n pair et les mélanges C11/C13, ou C13/C15 pour un nombre de carbone n impair.

Selon un mode préféré, on utilise un mélange d'au moins deux alcanes linéaires volatils comportant de 10 à 15 atomes de carbone distincts et différant entre eux d'un nombre de carbone d'au moins 2, et en particulier un mélange d'alcanes linéaires volatils C11/C13 ou un mélange d'alcanes linéaires volatils C12/C14.

D'autres mélanges associant plus de 2 alcanes linéaires volatils selon l'invention, tels que par exemple un mélange d'au moins 3 alcanes linéaires volatils comportant de 9 à 15 atomes de carbone distincts et différant entre eux d'un nombre de carbone d'au moins 1, font également partie de l'invention, mais les mélanges de 2 alcanes linéaires volatils selon l'invention sont préférés (mélanges binaires).

Selon un mode particulier de l'invention, dans un mélange d'alcanes linéaires volatils, l'alcane linéaire volatil ayant le nombre de carbone le plus petit est majoritaire dans le mélange.

A titre d'exemples de mélanges convenant à l'invention, on peut citer notamment les mélanges suivants :
- de 50 à 90% en poids, de préférence de 55 à 80% en poids, préférentiellement encore de 60 à 75% en poids d'alcane linéaire volatil en Cn
- de 10 à 50% en poids, de préférence de 20 à 45% en poids, de préférence de 24 à 40% en poids, d'alcane linéaire volatil en Cn+x avec x supérieur ou égal à 1 et de préférence x=1 ou x=2,
par rapport au poids total des alcanes dans ledit mélange.

En particulier, ledit mélange d'alcanes selon l'invention contient :
- moins de 2% en poids, de préférence moins de 1% en poids d'alcanes ramifiés,
- et/ou moins de 2% en poids, de préférence moins de 1% en poids d'alcanes aromatiques,
- et/ou moins de 2% en poids, de préférence moins de 1% en poids et préférentiellement moins de 0.1% en poids d'alcanes insaturés dans le mélange.

Plus particulièrement, un alcane linéaire volatil convenant à l'invention peut être mis en oeuvre sous la forme d'un mélange n-undécane/n-tridécane.

En particulier, on utilisera un mélange d'alcanes linéaires volatils comprenant:
- de 55 à 80% en poids, de préférence de 60 à 75% en poids d'alcane linéaire volatil en C11 (n-undécane)
- de 20 à 45% en poids, de préférence de 24 à 40% en poids d'alcane linéaire volatil en C13 (n-tridécane)
par rapport au poids total des alcanes dans ledit mélange.

Selon un mode de réalisation particulier, le mélange d'alcanes est un mélange n-undécane/n-tridécane. En particulier un tel mélange peut être obtenu selon l'exemple 1 ou l'exemple 2 du WO 2008/155059.

Selon une autre alternative, un alcane linéaire volatil convenant à l'invention peut être mis en oeuvre sous la forme d'un mélange n-dodécane/n-tétradécane.

Une composition de l'invention peut comprendre de 0,5 % à 95 % en poids d'alcanes linéaires volatils, en particulier de 1 à 90 % en poids d'alcanes linéaires volatils, et plus particulièrement de 5 à 90 % en poids d'alcanes linéaires volatils par rapport au poids total de la composition.

Le rapport en poids entre les alcanes linéaires volatils liquides et le copolymère à base de résine de silicone et de silicone fluide est supérieur ou égal à 2. Il est de préférence compris entre 2 et 100 et encore plus préférentiellement compris entre 2.5 et 50.

### Pigments

La composition de l'invention peut de plus contenir un ou plusieurs pigments.

Par pigment, on entend tous les pigments apportant de la couleur aux matières kératiniques. Leur solubilité dans l'eau à 25 °C et à pression atmosphérique (760 mmHg) est inférieure à 0,05 %, et de préférence inférieure à 0,01 %.

Les pigments qui peuvent être utilisés sont notamment choisis parmi les pigments organiques et / ou minéraux connus de la technique, notamment ceux qui sont décrits dans l'encyclopédie de technologie chimique de Kirk-Othmer et dans l'encyclopédie de chimie industrielle de Ullmann.

Ces pigments peuvent se présenter sous forme de poudre ou de pâte pigmentaire. Ils peuvent être enrobés ou non enrobés.

Les pigments peuvent par exemple être choisis parmi les pigments minéraux, les pigments organiques, les laques, les pigments à effets spéciaux tels que les nacres, les pigments métalliques ou les paillettes, et leurs mélanges.

Le pigment peut être un pigment minéral. Par pigment minéral, on entend tout pigment qui répond à la définition de l'encyclopédie Ullmann dans le chapitre pigment inorganique. On peut citer, parmi les pigments minéraux utiles dans la présente invention, les oxydes de fer ou de chrome, le violet de manganèse, le bleu outremer, l'hydrate de chrome et le bleu ferrique, l'oxyde de titane.

Le pigment peut être une particule métallique constituée d'un métal pur ou d'un alliage de métaux comprenant plus de 80% de métaux.

La particule métallique utile dans la composition de l'invention se présente avantageusement sous forme de « plaquettes ». Par « plaquettes », on désigne des particules dont le rapport de la plus grande dimension à la plus petite dimension, appelé facteur de forme, est supérieur ou égal à 5.

Par « dimensions », on désigne les dimensions données par la distribution granulométrique statistique à la moitié de la population, dite D50. La particule métallique a en particulier un facteur de forme supérieur ou égal à 8 et notamment à 10, et par exemple supérieur ou égal à 15.

La particule métallique peut être choisie parmi l'argent, l'aluminium, le fer, le chrome, le nickel, le molybdène, l'or, le cuivre, le zinc, l'étain, le magnésium, l'acier, le bronze, le titane et les alliages de ces métaux. De préférence la particule métallique est choisie parmi le cuivre, le zinc, l'aluminium, le titane, l'argent, l'or et les alliages de ces métaux. On utilise plus préférentiellement une particule métallique choisie parmi l'aluminium (présentant avantageusement une teneur en aluminium supérieure ou égale à 99%), le cuivre (présentant avantageusement une teneur en cuivre supérieure ou égale à 95%), le bronze (présentant de préférence une teneur en cuivre allant de 70 à 95% et une teneur en zinc allant de 5 à 30%).

Les particules métalliques ont par exemple selon leur plus grande dimension, une taille moyenne inférieure ou égale à 25 µm, en particulier inférieure ou égale à 10 µm.

Par « taille moyenne », on désigne la dimension donnée par la distribution granulométrique statistique à la moitié de la population, dite D50.

Lesdites particules ont généralement une épaisseur inférieure ou égale à 1 µm, notamment inférieure ou égale à 0,7 µm, en particulier inférieure ou égale à 0,5 µm.

A titre de particule métallique utilisable dans la composition selon l'invention, on peut citer des particules d'aluminium, telles que celles commercialisées sous les dénominations STARBRITE 2100 EAC® par la société SIBERLINE et METALURE® par la société ECKART. On peut également citer les poudres de bronze telles que celles commercialisées sous les dénominations Premior Super 8000 par la société Wolstenholme et sous les dénominations ROTHOFLEX, LITHOFLEX et STANDARD par la société ECKART avec par exemple les références STANDART Bronze Powder Offset 3000 Super Pale Gold (D50 3-5 µm) et LITHOFLEX XA 40-03 Rich Pale Gold (D50 3-5 µm). On peut aussi citer les particules d'alliage métallique comme des poudres de bronze enrobées de silice commercialisées sous la dénomination de VISIONAIRE Honey (taille 5-50 µm)et sous la dénomination de VISIONAIRE Amber (taille 5-50 µm) par la société Eckart ainsi que celles commercialisées sous la dénomination DOROLAN 08/0 Pale Gold (D50 7-9 µm), la poudre d'aluminium enrobée de SiO2 de commercialisée sous la référence VISIONAIRE Silver Sea (taille 5-50 µm) et les poudres de cuivre enrobée de SiO2 commercialisée sous la référence VISIONAIRE Cinnamon (taille 5-50 µm) et sous la référence VISIONAIRE Lava (taille 5-50 µm) par la société ECKART ainsi que celles commercialisées sous la dénomination DOROLAN 10/0 Copper (D50 9-11 µm).

Le pigment peut-être un pigment organique. Par pigment organique, on entend tout pigment qui répond à la définition de l'encyclopédie Ullmann dans le chapitre pigment organique. Le pigment organique peut notamment être choisi parmi les composés nitroso, nitro, azo, xanthène, quinoléine, anthraquinone, phtalocyanine, isoindolinone, isoindoline, quinacridone, périnone, pérylène, dicétopyrrolopyrrole, thioindigo, dioxazine, triphénylméthane, quinophtalone.

En particulier, les pigments organiques peuvent être choisis parmi le carmin, le noir de carbone, le noir d'aniline, le jaune azo, la quinacridone, le bleu de phtalocyanine, le rouge sorgho, les pigments bleus codifiés dans le Color Index sous les références CI 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les réfénces CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.

Les pigments conformes à l'invention peuvent aussi être sous forme de pigments composites tels qu'ils sont décrits dans le brevet EP 1 184 426. Ces pigments composites peuvent être composés notamment de particules comportant un noyau inorganique, au moins un liant assurant la fixation des pigments organiques sur le noyau, et au moins un pigment organique recouvrant au moins partiellement le noyau.

Le pigment organique peut aussi être une laque. Par laque, on entend les colorants adsorbés sur des particules insolubles, l'ensemble ainsi obtenu restant insoluble lors de l'utilisation.

Les substrats inorganiques sur lesquels sont adsorbés les colorants sont par exemple l'alumine, la silice, le borosilicate de calcium et de sodium ou le borosilicate de calcium et d'aluminium, et l'aluminium.

Parmi les colorants, on peut citer le carmin de cochenille. On peut également citer les colorants connus sous les dénominations suivantes : D & C Red 21 (CI 45 380), D & C Orange 5 (CI 45 370), D & C Red 27 (CI 45 410), D & C Orange 10 (CI 45 425), D & C Red 3 (CI 45 430), D & C Red 4 (CI 15 510), D & C Red 33 (CI 17 200), D & C Yellow 5 (CI 19 140), D & C Yellow 6 (CI 15 985), D & C Green (CI 61 570), D & C Yellow 1 O (CI 77 002), D & C Green 3 (CI 42 053), D & C Blue 1 (CI 42 090).

A titre d'exemples de laques, on peut citer le produit connu sous la dénomination suivante : D & C Red 7 (CI 15 850:1).

Le pigment peut aussi être un pigment à effets spéciaux. Par pigments à effets spéciaux, on entend les pigments qui créent d'une manière générale une apparence colorée (caractérisée par une certaine nuance, une certaine vivacité et une certaine clarté) non uniforme et changeante en fonction des conditions d'observation (lumière, température, angles d'observation...). Ils s'opposent par-là même aux pigments colorés qui procurent une teinte uniforme opaque, semi-transparente ou transparente classique.

Il existe plusieurs types de pigments à effets spéciaux, ceux à faible indice de réfraction tels que les pigments fluorescents, photochromes ou thermochromes, et ceux à plus fort indice de réfraction tels que les nacres ou les paillettes.

A titre d'exemples de pigments à effets spéciaux, on peut citer les pigments nacrés tels que le mica recouvert d'oxyde de titane, ou d'oxychlorure de bismuth, les pigments nacrés colorés tels que le mica recouvert d'oxyde de titane et d'oxydes de fer, le mica recouvert d'oxyde de fer, le mica recouvert d'oxyde de titane et notamment de bleu ferrique ou d'oxyde de chrome, le mica recouvert d'oxyde de titane et d'un pigment organique tel que défini précédemment ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

Les nacres peuvent plus particulièrement posséder une couleur ou un reflet jaune, rose, rouge, bronze, orangé, brun, or et/ou cuivré.

A titre illustratif des nacres pouvant être mises en oeuvre dans le cadre de la présente invention, on peut notamment citer les nacres de couleur or notamment commercialisées par la société ENGELHARD sous le nom Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona), par la société Eckart sous la dénomination Prestige Bronze et Prestige Soft Bronze et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) et par la société Eckart sous la dénomination Prestige Copper et Prestige soft Copper; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), Dark Blue (117324) (Colorona), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

En plus des nacres sur un support mica, on peut envisager les pigments multicouches basés sur des substrats synthétiques comme l'alumine, la silice, le borosilicate de calcium et de sodium ou le borosilicate de calcium et d'aluminium, et l'aluminium.

On peut également citer les pigments à effet interférentiel non fixés sur un substrat comme les cristaux liquides (Helicones HC de Wacker), les paillettes holographiques interférentielles (Geometric Pigments ou Spectra f/x de Spectratek). Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques et les quantum dots, commercialisés par exemple par la société Quantum Dots Corporation.

La variété des pigments qui peuvent être utilisés dans la présente invention permet d'obtenir une riche palette de couleurs, ainsi que des effets optiques particuliers tels que des effets métalliques, interférentiels.

La taille du pigment utilisé dans la composition cosmétique selon la présente invention est généralement comprise entre 10 nm et 200 µm, de préférence entre 20 nm et 80 µm, et plus préférentiellement entre 30 nm et 50 µm.

Les pigments peuvent être dispersés dans le produit grâce à un agent dispersant.

L'agent dispersant sert à protéger les particules dispersées contre leur agglomération ou floculation. Cet agent dispersant peut être un tensioactif, un oligomère, un polymère ou un mélange de plusieurs d'entre eux, portant une ou des fonctionnalités ayant une affinité forte pour la surface des particules à disperser. En particulier, ils peuvent s'accrocher physiquement ou chimiquement à la surface des pigments. Ces dispersants présentent, en outre, au moins un groupe fonctionnel compatible ou soluble dans le milieu continu. En particulier, on utilise les esters de l'acide hydroxy-12 stéarique en particulier et d'acide gras en C8 à C20 et de polyol comme le glycérol, la diglycérine, tel que le stéarate d'acide poly(12-hydroxystéarique) de poids moléculaire d'environ 750 g/mole tel que celui vendu sous le nom de Solsperse 21 000 par la société Avecia, le polygycéryl-2 dipolyhydroxystéarate (nom CTFA) vendu sous la référence Dehymyls PGPH par la société Henkel ou encore l'acide polyhydroxystéarique tel que celui vendu sous la référence Arlacel P100 par la société Uniqema et leurs mélanges.

Comme autre dispersant utilisable dans les compositions de l'invention, on peut citer les dérivés ammonium quaternaire d'acides gras polycondensés comme le Solsperse 17 000 vendu par la société Avecia, les mélanges de polydiméthylsiloxane / oxypropylène tels que ceux vendus par la société Dow Corning sous les références DC2-5185, DC2-5225 C.

Les pigments utilisés dans la composition cosmétique selon l'invention peuvent être traités en surface par un agent organique.

Ainsi les pigments préalablement traités en surface utiles dans le cadre de l'invention sont des pigments qui ont subi totalement ou partiellement un traitement de surface de nature chimique, électronique, électro-chimique, mécano-chimique ou mécanique, avec un agent organique tel que ceux qui sont décrits notamment dans Cosmetics and Toiletries, Février 1990, Vol. 105, p. 53-64 avant d'être dispersés dans la composition conforme à l'invention. Ces agents organiques peuvent être par exemple choisis parmi les acides aminés ; les cires, par exemple la cire de carnauba et la cire d'abeille ; les acides gras, les alcools gras et leurs dérivés, tels que l'acide stéarique, l'acide hydroxystéarique, l'alcool stéarylique, l'alcool hydroxystéarylique, l'acide laurique et leurs dérivés ; les tensio-actifs anioniques ; les lécithines ; les sels de sodium, potassium, magnésium, fer, titane, zinc ou aluminium d'acides gras, par exemple le stéarate ou laurate d'aluminium ; les alcoxydes métalliques ; les polysaccharides, par exemple le chitosane, la cellulose et ses dérivés ; le polyéthylène ; les polymères (méth)acryliques, par exemple les polyméthylmethacrylates ; les polymères et copolymères contenant des motifs acrylates ; les protéines ; les alcanoamines ; les composés siliconés, par exemple les silicones, les polydiméthylsiloxanes, les alcoxysilanes, les alkylsilanes, les siloxy-silicates ; les composés organiques fluorés, par exemple les perfluoroalkyle éthers ; les composés fluoro-siliconés.

Les pigments traités en surface utiles dans la composition cosmétique selon l'invention peuvent aussi avoir été traités par un mélange de ces composés et / ou avoir subi plusieurs traitements de surface.

Les pigments traités en surface utiles dans le cadre de la présente invention peuvent être préparés selon des techniques de traitement de surface bien connues de l'homme de l'art ou trouvés tels quels dans le commerce.

De préférence, les pigments traités en surface sont recouverts par une couche organique.

L'agent organique avec lequel sont traités les pigments peut être déposé sur les pigments par évaporation de solvant, réaction chimique entre les molécules de l'agent de surface ou création d'une liaison covalente entre l'agent de surface et les pigments.

Le traitement en surface peut ainsi être réalisé par exemple par réaction chimique d'un agent de surface avec la surface des pigments et création d'une liaison covalente entre l'agent de surface et les pigments ou les charges. Cette méthode est notamment décrite dans le brevet US 4 578 266.

De préférence, on utilisera un agent organique lié aux pigments de manière covalente.

L'agent pour le traitement de surface peut représenter de 0,1 à 50 % en poids du poids total des pigments traités en surface, de préférence de 0,5 à 30 % en poids, et encore plus préférentiellement de 1 à 10 % en poids.

Lorsqu'ils sont présents, la quantité de pigments est généralement comprise entre 0,1 à 40 % en poids, de préférence 0,5 à 20 % en poids du poids total de la composition.

### Autres solvants volatils

La composition selon l'inventon peut contenir d'autres solvants volatils afin de moduler la vitesse d'évaporation de la composition

Ce solvant volatil peut être un solvant organique non siliconé, un solvant organique siliconé autres que les alcanes linéaires volatils définis précédemment ou leurs mélanges.

A titre de solvant organique non siliconé volatil, on peut citer :
- les alcanols volatils en C₁-C₄ tels que l'éthanol, l'isopropanol ;
- les alcanes volatils non linéaires en C₅-C₇ tels que le 2,3-diméthylbutane, le 2,2-diméthylbutane, le 2-méthylpentane, le 3-méthylpentane ;
- les esters d'acides en C₁-C₂₀ liquides et d'alcools en C₁-C₈ volatils tels que l'acétate de méthyle, l'acétate de n-butyle, l'acétate d'éthyle, l'acétate de propyle, l'acétate d'isopentyle, le 3-éthoxypropionate d'éthyle ; on peut aussi citer les néopentanoate d'isohexyle ou d'isodecyle ;
- les cétones liquides à température ambiante et volatiles telles que la méthyléthylcétone, la méthylisobutylcétone, la diisobutylcétone, l'isophorone, la cyclohexanone, l'acétone ;
- les polyols volatils tels que le propylène glycol ;
- les éthers volatils tels que le diméthoxyméthane, le diéthoxyéthane, le diéthyléther ;
- les éthers de glycol volatils comme le 2-butoxyéthanol, le butyle diglycol, le monométhyléther de diéthylène glycol, le n-butyléther de propylène glycol, l'acétate de monométhyl éther de propylène glycol ;
- les huiles hydrocarbonées volatiles non linéaires telles que les huiles volatiles hydrocarbonées ramifiées ayant de 8 à 16 atomes de carbone et leurs mélanges, et notamment les alcanes ramifiés en C₈-C₁₆ comme les iso-alcanes (appelées aussi isoparaffines) en C₈-C₁₆, l'isododécane, l'isodécane, et par exemple les huiles vendues sous les noms commerciaux d'Isopars ou de Permetyls, et leurs mélanges.
- les perfluoroalcanes volatils en C₄-C₁₀ tels que le dodécafluoropentane, le tétradécafluorohexane, le décafluoropentane ;
- les perfluorocycloalkyles volatils tels que le perfluorométhylcyclopentane, le 1,3-perfluorodiméthylcyclohexane et le perfluorodecaline, vendus respectivement sous les dénominations de "Flutec PC1®", "Flutec PC3®" et "Flutec PC6®" par la Société F2 Chemicals, ainsi que le perfluorodiméthylcyclobutane et la perfluoromorpholine ;
- les composés fluoroalkyles ou hétérofluoroalkyles volatils répondant à la formule suivante :

   CH₃-(CH₂)ₙ-[Z]ₜ-X-CF₃
dans laquelle t est 0 ou 1 ; n est 0, 1, 2 ou 3 ; X est un radical perfluoroalkyle divalent, linéaire ou ramifié, ayant de 2 à 5 atomes de carbone, et Z représente O, S, ou NR, R étant un atome d'hydrogène, un radical -(CH₂)ₙ-CH₃ ou un radical -(CF₂)ₘ-CF₃, m étant 2, 3, 4 ou 5.

Parmi les composés fluoroalkyles ou hétérofluoroalkyles volatils, on peut notamment citer le méthoxynonafluorobutane vendu sous la dénomination de "MSX 4518®", "HFE-7100®" par la Société 3M et l'éthoxynonafluorobutane vendu sous la dénomination de "HFE-7200®" par la Société 3M.

De préférence, le solvant est choisi de telle manière que son point d'ébullition soit inférieur à 200 °C.

Selon un mode de réalisation particulier, le solvant organique non siliconé additionnel est choisi parmi l'éthanol, l'isopropanol, l'acétone, les alcanes non linéaires liquides à 25 °C et à pression atmosphérique (760 mmHg) tels que l'isododécane.

A titre de solvant siliconé volatil, on peut citer les composés siliconés à faible viscosité choisi parmi les silicones linéaires ou cycliques ayant de 2 à 7 atomes de silicium, ces silicones comportant éventuellement des groupes alkyle ou alkoxy ayant de 1 à 10 atomes de carbone, par exemple l'octaméthylcyclotétrasiloxane, la décaméthylcyclopentasiloxane, la dodécaméthylcyclohexasiloxane, l'heptaméthylhexyltri-siloxane, l'heptaméthyléthyltrisiloxane, l'heptaméthyloctyltrisiloxane, l'octaméthyltrisiloxane, la décaméthyltétrasiloxane, et leurs mélanges. Selon un mode de réalisation particulier, le composé siliconé est choisi parmi la cyclopentadiméthylsiloxane et la dodécaméthylcyclohexasiloxane.

Selon un mode de réalisation particulier, le solvant siliconé volatil présente une viscosité inférieure à 50 centistokes.

De préférence, la silicone volatile est choisie parmi la décaméthylcyclopentasiloxane, la dodécaméthylcyclohexasiloxane, l'octaméthyltrisiloxane et la décaméthyltétrasiloxane.

A titre d'exemple, on peut citer la décaméthylcyclopentasiloxane commercialisée sous le nom DC-245 par la société Dow Corning, la dodécaméthylcyclohexasiloxane commercialisée sous le nom DC-246 par la société Dow Corning, l'octaméthyltrisiloxane commercialisée sous le nom DC-200 Fluid 1 cst par la société Dow Corning et la décaméthyltétrasiloxane commercialisée sous le nom DC-200 Fluid 1,5 cst par la société Dow Corning.

Selon un mode de réalisation particulier de l'invention, le ou les solvants volatils sont choisis parmi l'eau, l'éthanol, l'isopropanol, l'acétone, l'isododécane, la décaméthylcyclopentasiloxane, la dodécaméthylcyclohexasiloxane, l'octaméthyltrisiloxane et la décaméthyltétrasiloxane et leurs mélanges.

Le solvant volatil peut être présent dans la composition utile dans le procédé de l'invention en une teneur allant de 0,1 % à 95 % en poids par rapport au poids total de la composition, de préférence allant de 1 % à 90 % en poids, et préférentiellement allant de 5 % à 90 % en poids.

### Additifs additionels

Selon un mode de réalisation particulier, la composition de l'invention peut contenir un ou plusieurs polysiloxanes présentant une viscosité supérieure à 100 cst, préférentiellement supérieure à 300 cst. La viscosité de ces polysiloxanes peut être mesurée selon la norme ASTM D-445. De tels polysiloxanes peuvent être des huiles, des gommes ou des résines de silicone, les silicones réticulées.

A titre de polysiloxanes de viscosité supérieure à 100 cst, on peut notamment citer les polydiméthylsiloxanes; les alkyldiméthicones; les polyphénylméthylsiloxanes tels que les phényldiméthicones, les phényltriméthicones, et les vinylméthylméthicones; ainsi que les silicones modifiées par des groupements aliphatiques et / ou aromatiques, éventuellement fluorés, ou par des groupements fonctionnels tels que des groupements hydroxyles, thiols et / ou amines.

De tels polysiloxanes peuvent choisis parmi les silicones de formule (I) : 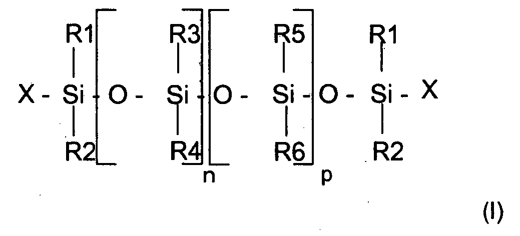 dans laquelle :
R₁, R₂, R₅ et R₆ sont, ensemble ou séparément, un radical alkyle ayant 1 à 6 atomes de carbone, R₃ et R₄ sont, ensemble ou séparément, un radical alkyle ayant de 1 à 6 atomes de carbone, un radical vinyle, un radical aryle, un radical aminoalkyle ayant de 1 à 6 atomes de carbone éventuellement substitué, un radical hydroxyle, un radical thioalkyle ayant de 1 à 6 atomes de carbone, X est un radical alkyle ayant de 1 à 6 atomes de carbone, un radical hydroxyle, un radical vinyle, un radical aminoalkyle ayant de 1 à 6 atomes de carbone éventuellement substitué, un radical thioalkyle ayant de 1 à 6 atomes de carbone, n et p étant des entiers choisis de manière à obtenir une viscosité supérieure à 300 cst.

A titre d'exemple, on peut citer les polydiméthylsiloxanes suivantes :
- les substituants R₁ à R₆ et X représentent un groupement méthyle, comme celle vendue sous la dénomination Baysilicone TP 3898 par la société Général Electric, et celle vendue sous la dénomination AK 500000 par la société Waker,
- les substituants R₁ à R₆ et X représentent un groupement méthyle, p et n sont tels que le poids moléculaire est de 120 000 g/mol, comme celle vendue sous la dénomination Dow Corning 200 Fluid 60000 CS par la société Dow Corning,
- les substituants R₁ à R₆ et X représentent un groupement méthyle, p et n sont tels que le poids moléculaire est de 250 000 g/mol, comme celle vendue sous la dénomination Mirasil DM 500.000 par la société Rhodia et celle vendue sous la dénomination Dow Corning 200 Fluid 500.000 cst par la société Dow Corning,
- les substituants R₁ à R₆ représentent un groupement méthyle, le groupement X représente un groupement hydroxy, n et p sont tels que le poids moléculaire du polymère soit de 600 000 g/mol, comme celle vendue sous la dénomination SGM 36 par la société Dow Corning,
- les diméthicones du type (polydiméthylsiloxane)(méthylvinylsiloxane) telle que la SE63 commercialisée par GE BAYER Silicones, les copolymères poly(diméthylsiloxane)(diphényl)(méthylvinylsiloxane), et leurs mélanges.

Dans le cas où le polysiloxane comprend un groupement fluoré, on peut choisir les copolymères de structure suivante : 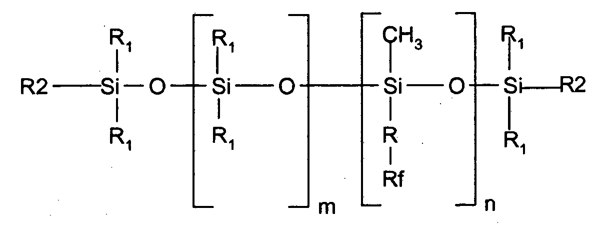 dans laquelle :
R représente un groupement divalent alkyle linéaire ou ramifié, ayant 1 à 6 atomes de carbone, de préférence un groupement divalent méthyle, éthyle, propyle ou butyle, Rf représente un radical fluoroalkyle, notamment un radical perfluoroalkyle, ayant 1 à 12 atomes de carbone, de préférence 1 à 9 atomes de carbone, R₁ représente, indépendamment l'un de l'autre, un radical alkyle en C₁-C₂₀, un radical hydroxyle, un radical phényle, R₂ représente R₁ ou R_{f},
m est choisi de 0 à 500, de préférence de 0 à 200, et n est choisi de 1 à 1000, de préférence de 1 à 500.

De préférence, les groupements R1 sont identiques et représentent un radical méthyle.

De tels polysiloxanes sont notamment ceux commercialisés par la société Shin Etsu sous les dénominations 'FL-5', 'FL-10', 'X22-82l' et 'X22-822' ou encore 'FL-100', par la société Dow Corning sous le nom FS-1265 Fluid, par la société Phoenix Chemical sous la gamme Pecosil FS sous les dénominations Pecosil FSL-150, Pecosil FSL-300, Pecosil FSH-150, Pecosil FSH-300, Pecosil FSU-150, Pecosil FSU-300.

La masse moléculaire en poids du ou des polysiloxane peut être comprise entre 1000 et 1 500 000 g/mol, notamment entre 20 000 et 1 000 000 g/mol.

Le polysiloxane peut être sous forme de résine. Par le terme « résine », on entend une structure tridimensionnelle réticulée ou non. A titre d'exemple de résine de polysiloxane, on peut citer les silsesquioxanes et les siloxysilicates.

La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.

La lettre M représente l'unité monofonctionelle de formule (CH₃)₃SiO_{1/2}, l'atome de silicium étant relié à un seul atome d'oxygène dans le polymère comprenant cette unité.

La lettre D signifie une unité difonctionnelle (CH₃)₂SiO_{2/2} dans laquelle l'atome de silicium est relié à deux atomes d'oxygène

La lettre T représente une unité trifonctionnelle de formule (CHₛ)SiO_{3/2}.

Dans les motifs M, D, T définis précédemment, au moins un des groupes méthyles peut être substitués par un groupe R différent du groupe méthyle tel qu'un radical hydrocarboné (notamment alkyle) ayant de 2 à 10 atomes de carbone ou un groupe phényl ou bien encore un groupe hydroxyle.

Enfin, la lettre Q signifie une unité tetrafonctionnelle SiO_{4/2} dans laquelle l'atome de silicium est lié à quatre atomes d'hydrogène eux mêmes liés au reste du polymère.

Divers résines de propriétés différentes peuvent être obtenues à partir de ces différentes unités, les propriétés des ces polymères variant en fonction du type de monomères (ou unités), du type et du nombre de radicaux subsitués, de la longueur de la chaîne polymérique, du degré de ramification et de la taille des chaines pendantes.

A titre d'exemple de ces résines silicones, on peut citer :
- les siloxysilicates qui peuvent être des triméthylsiloxysilicate de formule [(CH₃)₃SiO]ₓ(SiO_{4/2})_{y} (unités MQ) dans laquelle x et y sont des entiers allant de 50 à 80,
   - les polysilesquioxanes de formule (CH₃SiO_{3/2})ₓ (unités T) dans laquelle au moins un des radicaux méthyle peut être substitué par un groupement R tel que défini plus haut. De préférence le nombre x d'unités T du silsesquioxane est inférieur ou égal à 500, il est plus préférentiellement compris entre 50 et 500. Le poids moléculaire de la résine de silicone selon l'invention est donc de préférence compris entre 500 et 50 000 g/mol, plus préférentiellement compris entre 500 et 20 000 g/mol et encore plus préférentiellement compris entre 500 et 10 000 g/mol ;
- les polyméthylsilsesquioxanes qui sont des polysilsesquioxanes dans lesquels aucun des radicaux méthyle n'est substitué par un autre groupement. De tels polymethylsilsesquioxanes sont décrits dans le document US 5 246 694 dont le contenu est incorporé par référence ;
- les polypropylsilsesquioxanes, pour lesquelles les radicaux méthyle sont remplacés par des radicaux propyle. Ces composés ainsi que leur synthèse sont notamment décrit dans la demande de brevet WO 2005/075567 ;
- les polyphénylsilsesquioxanes, pour lesquelles les radicaux méthyle sont remplacés par des radicaux phényle. Ces composés ainsi que leur synthèse sont notamment décrit dans la demande de brevet US 2004/0180011.

A titre d'exemples de résines polyméthylsilsesquioxanes commercialement disponibles, on peut citer celles qui sont commercialisées :
- par la société Wacker sous la référence Resin MK tels que la Belsil PMS MK : polymère comprenant des unités répétitives CH₃SiO_{3/2} (unités T), pouvant aussi comprendre jusqu'à 1 % en poids d'unités (CH₃)₂SiO_{2/2} (unités D) et présentant un poids moléculaire moyen d'environ 10 000 g/mol. On pense que le polymère se trouve dans une configuration « cage » et « échelle » comme cela est représenté dans les figures ci-dessous. Le poids moléculaire moyen des unités en configuration « cage » a été calculé à 536 g/mol. La majorité du polymère se trouve en configuration « échelle » avec des groupes éthoxy aux extrémités. Ces groupes éthoxy représentent 4,5 % en masse du polymère. Comme ces extrémités peuvent réagir avec l'eau, un taux faible et variable de groupes SiOH peut également être présent. 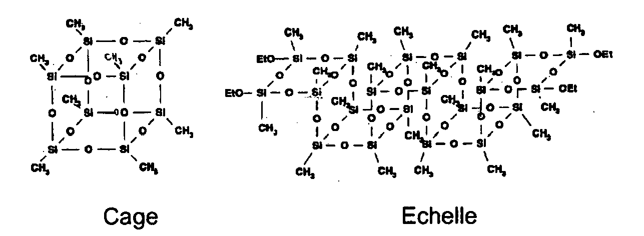
- par la société SHIN-ETSU sous les références KR-220L qui sont composées d'unités T de formule CH₃SiO_{3/2} et ont des groupes terminaux Si-OH (silanol), sous la référence KR-242A qui comprennent 98 % d'unités T et 2 % d'unités diméthyle D et ont des groupes terminaux Si-OH ou encore sous la référence KR-251 comprenant 88 % d'unités T et 12 % d'unités diméthyle D et ont des groupes terminaux Si-OH.

A titre d'exemples de résines polypropylsilsesquioxanes commercialement disponibles, on peut citer celles qui sont commercialisées :
- par la société DOW CORNING sous la référence Dow Corning 670 Fluid qui est une polypropylsilsesquioxane diluée dans la D5.

A titre d'exemples de résines polyphénylsilsesquioxanes commercialement disponibles, on peut citer celles qui sont commercialisées :
- par la société DOW CORNING sous la référence Dow Corning 217 Flake Resin qui est une polyphénylsilsesquioxane aux extrémités silanol ;
- par la société WACKER sous la référence Belsil SPR 45 VP.

Comme résines siloxysilicates, on peut citer les résines triméthylsiloxysilicate (TMS) éventuellement sous forme de poudres. De telles résines sont commercialisées sous la référence SR1000 par la société General Electric ou sous la référence TMS 803 par la société Wacker. On peut encore citer les résines timéthylsiloxysilicate commercialisées dans un solvant tel que la cyclométhicone, vendues sous la dénomination "KF-7312J" par la société Shin-Etsu, "DC 749", "DC 593" par la société Dow Corning.

La résine de silicone selon l'invention est de préférence filmogène. En effet, tous les silsesquioxanes ne sont pas filmogènes, par exemple les polyméthylsilsesquioxanes hautement polymérisées comme les TospearITM de Toshiba ou la KMP590 de Shin-Etsu sont insolubles et ne sont pas filmogènes.

Dans un mode de réalisation de l'invention, la ou les résines de silicones sont solubles ou dispersables dans la composition de l'invention. De préférence, les résines de silicone selon l'invention sont solubles dans les silicones volatiles et les solvants organiques. Dans un mode de réalisation, la résine de silicone est solide à 25 °C.

Les résines de silicones préférées selon l'invention sont les résines triméthylsiloxysilicates, les résines polyméthylsilsesquioxanes et les résines polypropylsilsesquioxanes.

La composition de l'invention peut aussi contenir une silicone réticulée telle qu'un organopolysiloxane élastomère réticulé, un composé siliconé de haut poids moléculaire présentant une structure tridimensionnelle, aux propriétés viscoélastiques d'un matériau solide souple. Ces organopolysiloxanes peuvent ainsi se présenter sous forme sèche en poudre, ou sous forme gonflée, dans un solvant, le produit résultant étant généralement un gel. Ces produits peuvent également se présenter sous forme dispersée dans une solution aqueuse.

La synthèse de ces organopolysiloxane est décrite dans les brevets suivants :
- US 5 266 321 de Kobayashi Kose,
- US 4 742 142 de Toray Silicone,
- US 5 654 362 de Dow Corning Corp,
- la demande de brevet FR 2 864 784.

Les organopolysiloxanes élastomères utilisés dans la composition peuvent être partiellement ou totalement réticulés. Ils se présentent généralement sous forme de particules. En particulier, les particules d'organopolysiloxane élastomère ont une taille moyenne en nombre allant de 0,1 à 500 µm,. Ces particules peuvent avoir toute forme et par exemple être sphériques, plates ou amorphes.

L'organopolysiloxane réticulé obtenu peut être un composé non-émulsionnant ou un composé émulsionnant. Le terme "non émulsionnant" défini des organopolysiloxanes réticulés ne contenant pas de motifs polyoxyalkylène. Le terme "émulsionnant" signifie des composés organopoysiloxanes réticulés ayant au moins un motif polyoxyalkylène, notamment polyoxyéthylène ou polyoxypropylène.

Les particules d'organopolysiloxane réticulé peuvent être véhiculées sous forme de gel constitué d'un organopolysiloxane réticulé inclus dans au moins une huile hydrocarbonée et / ou une huile siliconée. Dans ces gels, les particules d'organopolysiloxane sont souvent des particules non-sphériques. Les particules d'organopolysiloxane réticulé peuvent également se présenter sous forme de poudre, notamment sous forme de poudre sphérique.

Des organopolysiloxanes réticulés non-émulsionnants sont notamment décrits dans les brevets US 4 970 252, US 4 987 169, US 5 412 004, US 5 654 362, US 5 760 116, dans la demande JP-A-61-194009.

Comme organopolysiloxanes réticulés non-émulsionnants, on peut utiliser ceux vendus sous les dénominations "KSG-6", "KSG-15", "KSG-16", "KSG-18", "KSG-31", "KSG-32", "KSG-33", "KSG-41", "KSG-42", "KSG-43", "KSG-44" « USG-103 » par la société Shin Etsu, "DC 9040", "DC9041", "DC 9509", "DC9505", "DC 9506" « DC 9045 » par la société Dow Corning, "GRANSIL" par la société Grant Industries, "SFE 839" par la société General Electric.

Avantageusement, les organopolysiloxanes réticulés émulsionnants comprennent les organopolysiloxanes modifiés polyoxyalkylène formé à partir de composés divinyliques, en particulier des polysiloxanes ayant au moins deux groupes vinyliques, réagissant avec des liaisons Si-H d'un polysiloxane. Des organopolysiloxanes réticulés émulsionnants sont notamment décrits dans les brevets US 5 236 986, US 5 412 004, US 5 837 793, US 5 811 487.

Comme organopolysiloxanes réticulés émulsionnants, on peut utiliser ceux commercialisés sous les dénominations "KSG-21 ", "KSG-20", "KSG-30"par la société Shin Etsu, et "DC9010", "DC9011" par la société Dow Corning.

Les particules d'organopolysiloxane réticulé élastomère peuvent se présenter également sous forme de poudre d'organopolysiloxane réticulé élastomère enrobée de résine de silicone, notamment de résine silsesquioxane, comme décrit par exemple dans le brevet US 5 538 793.

De tels élastomères sont vendus sous les dénomination "KSP-100", "KSP-101 ", "KSP-102", "KSP-103", KSP-104", "KSP-105" par la société Shin Etsu.

De préférence l'organopolysiloxane réticulé est non-émulsionnant.

La composition de l'invention peut aussi contenir un polymère siliconé greffé. Dans le cadre de l'invention, on entend par polymère siliconé greffé, un polymère comprenant une portion polysiloxane et une portion constituée d'une chaîne organique non-siliconée, l'une des deux portions constituant la chaîne principale du polymère l'autre étant greffée sur ladite chaîne principale.

Les polymères siliconés greffés utilisés dans la composition cosmétique selon l'invention sont choisis préférentiellement dans le groupe constitué par les polymères à squelette organique non siliconé greffé par des monomères contenant un polysiloxane, les polymères à squelette polysiloxanique greffé par des monomères organiques non siliconés et leurs mélanges.

Les monomères organiques non siliconés constituant la chaîne principale du polymère siliconé greffé peuvent être choisis parmi des monomères à insaturation éthylénique polymérisables par voie radicalaire, des monomères polymérisables par polycondensation tels que ceux formant des polyamides, des polyesters, des polyuréthanes, des monomères à ouverture de cycle tels que ceux du type oxazoline ou caprolactone.

Les polymères à squelette organique non-siliconé greffé par des monomères contenant un polysiloxane, conformes à l'invention, peuvent être choisis parmi ceux décrits dans les brevets US 4,693,935, US 4,728,571 et US 4,972,037 et les demandes de brevet EP-A-0 412 704, EP-A-0 412 707, EP-A-0 640 105 et WO 95/00578. Il s'agit de copolymères obtenus par polymérisation radicalaire à partir de monomères à insaturation éthylénique et de macromères siliconés ayant un groupe vinylique terminal ou bien des copolymères obtenus par réaction d'une polyoléfine comprenant des groupes fonctionnalisés et d'un macromère polysiloxane ayant une fonction terminale réactive avec lesdits groupes fonctionnalisés.

Le copolymère à squelette organique non-siliconé greffé par des monomères contenant un polysiloxane peut par exemple avoir la structure suivante :

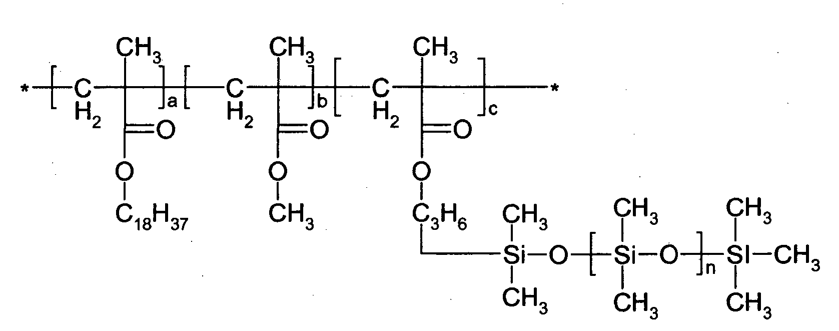

Un tel polymère est commercialisé sous le nom KP 561 commercialisés par Shin Etsu.

Le copolymère à squelette organique non-siliconé greffé par des monomères contenant un polysiloxane peut aussi avoir la structure suivante :

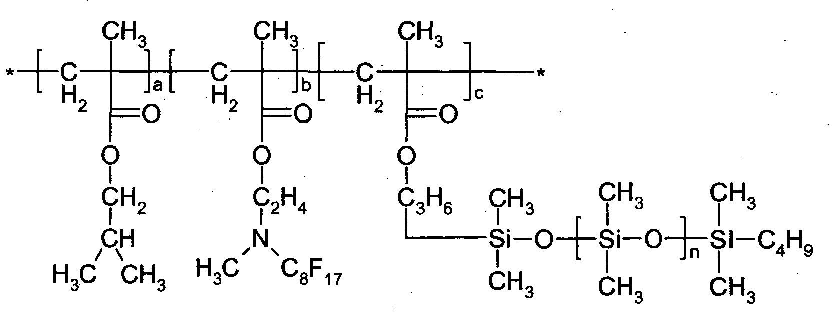

Un tel polymère, le Polysilicone 7, est commercialisé sous le nom SA70 par 3M.

D'autres copolymères à squelette organique non-siliconé greffé par des monomères contenant un polysiloxane peuvent aussi être le KP545, le KP574 et le KP575 commercialisés par Shin Etsu.

Comme composé siliconé greffé, on peut également citer le copolymère d'isobutylmethacrylate / bis-hydroxypropyl dimethicone acrylate commercialisé par Grant Industrie sous le nom Granacrysil BMAS.

Selon la présente invention, le ou les polymères siliconés greffés, à squelette polysiloxanique greffé par des monomères organiques non siliconés comprennent une chaîne principale de silicone (ou polysiloxane (≡Si-O-)ₙ) sur laquelle se trouve greffé, à l'intérieur de ladite chaîne ainsi qu'éventuellement à l'une au moins de ses extrémités, au moins un groupement organique ne comportant pas de silicone.

Des exemples de polymères siliconés correspondant à la définition sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle. Comme composé répondant à cette définition on peut citer le poly dimethyl / methyl siloxane à groupements propyl thio-3 acrylate de methyle / methacrylate de methyle / acide methacrylique ou Polysilicone-8 commercialisé sous le nom VS80 par la société 3M.

D'autres exemples de polymères siliconés sont notamment des polydiméthylsiloxanes (PDMS) sur lesquels sont greffés, par l'intermédiaire d'un chaînon de raccordement de type thiopropylène, des motifs polymères du type poly(méth)acrylate d'isobutyle.

De préférence, la masse moléculaire en nombre des polymères siliconés à squelette polysiloxanique greffé par des monomères organiques non siliconés de l'invention varie de 10 000 à 1 000 000 environ, et encore plus préférentiellement de 10 000 à 100 000 environ.

De préférence, les polymères siliconés greffés sont choisis dans le groupe constitué par le copolymère de méthacrylate d'alkyles greffé polydiméthylsiloxane, les copolymères de méthacrylate d'isobutyle, d'acide acrylique et de macromère siliconé et le poly diméthyl / méthyl siloxane à groupements propyl thio-3-acrylate de méthyle / méthacrylate de méthyle / acide méthacrylique.

Les composés siliconés préférés sont les huiles siliconées, notamment celles décrites dans la formule (I), et les résines de silicone.

Lorsqu'ils sont présents dans la composition conforme à l'invention, la quantité en composés siliconés additionnels est comprise entre 0,1 et 30 % en poids, de préférence entre 0,1 et 20 % en poids, et encore plus préférentiellement entre 0,1 et 10 % en poids du poids total de la composition.

La composition selon l'invention peut comprendre un ou plusieurs agents épaississants choisis parmi les agents épaississants polymériques, les agents épaississants minéraux et leurs mélanges.

L'épaississant peut être minéral ou organique, polymère ou non polymère. L'épaississant peut être choisi pour épaissir une phase aqueuse ou une phase grasse de la composition selon les cas.

On entend par épaississant, un composé qui modifie la rhéologie du milieu dans lequel il est incorporé en accroissant d'au moins 100 cps la viscosité du milieu à 25 °C et à un taux de cisaillement de 1 s⁻¹. Cette viscosité peut être mesurée à l'aide d'un viscosimètre cône/plan (Rhéomètre Haake R600 ou analogue).

L'épaississant de milieu aqueux peut être choisi parmi :
- les argiles hydrophiles,
- la silice pyrogénée hydrophile,
- les épaississants cellulosiques hydrosolubles, tels que l'hydroxyethylcellulose, la méthylcellulose, l'hydroxypropylcellulose. Parmi celles-ci on peut citer notamment les gommes vendues sous la dénomination Cellolize QP 4400 H par a société Amercol.
- les gommes de guar non ionique comprenant des groupements hydroxyalkyle en C₁-C₆. On peut mentionner à titre d'exemple les groupements hydroxymethyle, hydroxypropyle et hydroxybutyle. De telles gommes de guar sont notamment vendue sous les dénominations commerciales JAGUAR HP8, JAGUAR HP60, JAGUAR HP120, JAGUAR HP105 par la société MEYHALL ou sous la dénomination GALACTASOL 40H4FD2 par la société AQUALON.
- les carraghénanes,
- les gommes de caroube, de scléroglucane, de gellane, de rhamsan, de karaya,
- les alginates, les maltodextrines, l'amidon et ses dérivés, l'acide hyaluronique et ses sels,
- les polymères poly(métha)crylates de glycéryle vendus sous les dénominations de "Hispagel" ou "Lubragel" par les Sociétés Hispano Quimica ou Guardian,
- l'alcool polyvinylique,
- les polymères et les copolymères réticulés d'acrylamide, tels que ceux vendus sous les dénominations de "PAS 5161" ou "Bozepol C" par la Société Hoechst, de "Sepigel 305" par la Société Seppic par la Société Allied Colloïd, ou encore,
- les homopolymères réticulés de chlorure de méthacryloyloxyéthyltriméthylammonium vendus sous la dénomination de "Salcare SC95" par la Société Allied Colloïd,
- les polymères associatifs et notamment les polyuréthanes associatifs.

De tels épaississants sont notamment décrits dans les demandes EP-A-1400234, dont le contenu est incorporé à titre de référence.

L'épaississant de milieu huileux peut être choisi parmi
- les argiles organophiles,
- les silices pyrogénées hydrophobes
- les gommes de guar alkylées (avec groupe alkyle en C1-C6), telles que celles décrites dans EP-A-708114 ;
- les polymères gélifiant d'huile comme les polymères tribloc ou en étoile résultant de la polymérisation ou copolymérisation d'au moins monomère à groupe éthylénique, comme les polymères vendus sous la dénomination Kraton ;
- les polymères de masse moléculaire moyenne en poids inférieure à 100 000, comportant a) un squelette polymérique ayant des motifs de répétition hydrocarbonés pourvus d'au moins un hétéroatome, et éventuellement b) au moins une chaîne grasse pendante et / ou au moins une chaîne grasse terminale éventuellement fonctionnalisées, ayant de 6 à 120 atomes de carbone et étant liées à ces motifs hydrocarbonés, telles que décrites dans les demandes WO-A-02/056847, WO-A-02/47619 dont le contenu est incorporé à titre de référence ; en particulier les résines de polyamides (notamment comprenant des groupes alkyles ayant de 12 à 22 atomes de carbone) telles que celles décrites dans US-A-5783657 dont le contenu est incorporé à titre de référence.
- les résines de polyamides siliconées telles que décrites dans la demande EP-A-1266647, dans la demande de brevet français déposée sous le n° 0 216 039 dont le contenu est incorporé à titre de référence.

De tels épaississants sont notamment décrits dans les demandes EP-A-1400234, dont le contenu est incorporé à titre de référence.

L'épaississant peut être un agent gélifiant organique, c'est-à-dire un agent comprenant au moins un composé organique. Les organogélateurs peuvent être choisis parmi ceux décrits dans la demande WO-A-03/105788 dont le contenu est incorporé à titre de référence.

Plus précisément, l'agent épaississant polymérique présent dans la composition selon l'invention est un polymère amorphe formé par polymérisation d'une oléfine. L'oléfine peut être notamment un monomère à insaturation éthylénique élastomérique.

Comme exemple d'oléfine, on peut citer les monomères de carbure éthylénique, ayant notamment une ou deux insaturations éthylénique, ayant de 2 à 5 atomes de carbone tels que l'éthylène, le propylène, le butadiène, l'isoprène.

L'agent épaississant polymérique est capable d'épaissir ou de gélifier la composition. Par polymère amorphe, on entend un polymère qui n'a pas de forme cristalline. L'agent épaississant polymérique peut être également filmogène.

L'agent épaississant polymérique peut être notamment un copolymère dibloc, tribloc, multibloc, radial, étoile, ou leurs mélanges.

De tels agents épaississants polymériques sont décrits dans la demande US-A-2002/005562 et dans le brevet US-A-5 221 534

Avantageusement, l'agent épaississant polymérique est un copolymère bloc amorphe de styrène et d'oléfine.

L'agent épaississant polymérique est de préférence hydrogéné pour réduire les insaturations éthyléniques résiduelles après la polymérisation des monomères.

En particulier, l'agent épaississant polymérique est un copolymère, éventuellement hydrogéné, à blocs styrène et à blocs éthylène/alkylène en C3-C4.

Comme copolymère dibloc, de préférence hydrogéné, on peut citer les copolymères de styrène-éthylène/propylène, les copolymères de styrène-éthylène/butadiène. Des polymères diblocs sont notamment vendus sous la dénomination Kraton® G1701 E par la société Kraton Polymers.

Comme copolymère tribloc, de préférence hydrogéné, on peut citer les copolymères de styrène-éthylène/propylène-styrène, les copolymères de styrène-éthylène/butadiène-styrène, les copolymères de styrène-isoprène-styrène, les copolymères de styrène-butadiène-styrène. Des polymères triblocs sont notamment vendus sous les dénominations Kraton® G1650, Kraton® G1652, Kraton® D1101, Kraton® D1102, Kraton® D1160 par la société Kraton Polymers.

On peut également utiliser un mélange de copolymère hydrogéné tribloc styrène-butylène / éthylène-styrène et de polymère étoile hydrogéné éthylènepropylène-styrène, un tel mélange étant notamment dans l'isododécane. De tels mélanges sont par exemple vendus par la société PENRECO sous les dénominations commerciales VERSAGEL® M5960 et VERSAGEL® M5670.

Avantageusement, on utilise comme agent épaississant polymérique un copolymère dibloc tel que ceux décrits précédemment, en particulier un copolymère dibloc de styrène-éthylène / propylène.

Plus précisément, les argiles organophiles sont des argiles modifiées par des composés chimiques rendant l'argile apte à gonfler.

Les argiles sont des produits déjà bien connus en soi, qui sont décrits par exemple dans l'ouvrage "Minéralogie des argiles, S. Caillère, S. Hénin, M. Rautureau, 2ème édition 1982, Masson", dont l'enseignement est ici inclus à titre de référence.

Les argiles sont des silicates contenant un cation pouvant être choisi parmi les cations de calcium, de magnésium, d'aluminium, de sodium, de potassium, de lithium et leurs mélanges.

A titre d'exemples de tels produits, on peut citer les argiles de la famille des smectites telles que les montmorillonites, les hectorites, les bentonites, les beidellites, les saponites, ainsi que de la famille des vermiculites, de la stévensite, des chlorites.

Ces argiles peuvent être d'origine naturelle ou synthétique. De préférence, on utilise les argiles qui sont cosmétiquement compatibles et acceptables avec les matières kératiniques.

L'argile organophile peut être choisie parmi la montmorrilonite, la bentonite, l'hectorite, l'attapulgite, la sépiolite, et leurs mélanges. L'argile est de préférence une bentonite ou une hectorite.

Ces argiles peuvent être modifiées avec un composé chimique choisi parmi les amines quaternaires, les amines tertiaires, les acétates aminés, les imidazolines, les savons aminés, les sulfates gras, les alkyl aryl sulfonates, les oxides amines, et leurs mélanges.

Comme argiles organophiles, on peut citer les quaternium-18 bentonites telles que celles vendues sous les dénominations Bentone 3, Bentone 38, Bentone 38V par la société Rhéox, Tixogel VP par la société United catalyst, Claytone 34, Claytone 40, Claytone XL par la société Southern Clay; les stéaralkonium bentonites telles que celles vendues sous les dénominations Bentone 27 par la société Rheox, Tixogel LG par la société United Catalyst, Claytone AF, Claytone APA par la société Southern Clay ; les quaternium-18/benzalkonium bentonite telles que celles vendues sous les dénominations Claytone HT, Claytone PS par la société Southern Clay.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leurs surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot.

Il est possible de modifier chimiquement la surface de ladite silice, par réaction chimique générant une diminution du nombre de groupes silanol. On peut notamment substituer des groupes silanol par des groupements hydrophobes : on obtient alors une silice hydrophobe.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6ème édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

La silice pyrogénée présente de préférence une taille de particules pouvant être nanométrique à micrométrique, par exemple allant d'environ de 5 à 200 nm.

De préférence, on utilise comme agent épaississant minéral une hectorite ou une bentonite organomodifiée.

Le ou les agents épaississants peuvent être présents dans la composition en une teneur totale allant de 0,1 à 10 % en poids par rapport au poids total de la composition, de préférence allant de 0,5 à 7 % en poids, et plus préférentiellement allant de 0,5 à 4 % en poids.

Les compositions conformes à l'invention peuvent également contenir un ou plusieurs agents utilisés habituellement en cosmétique, choisi, par exemple, parmi des agents réducteurs, des corps gras, des plastifiants, des adoucissants, des agents anti-mousse, des agents hydratants, des filtres UV, des colloïdes minéraux, des peptisants, des solubilisants, des parfums, des protéines, des vitamines, des propulseurs, les cires oxyéthylénées ou non, les paraffines, les acides gras en C₁₀-C₃₀ tels que l'acide stéarique, l'acide laurique, les amides gras en C₁₀-C₃₀ tel que le diéthanolamide laurique.

Les additifs ci dessus sont en général présents en quantité comprise pour chacun d'eux entre 0,01 et 20 % en poids par rapport au poids de la composition.

Bien entendu, l'homme de l'art veillera à choisir ce ou ces éventuels additifs de manière telle que les propriétés avantageuses attachées intrinsèquement à la formation du gainage conforme à l'invention ne soient pas, ou substantiellement pas, altérées.

La composition selon l'invention peut se présenter notamment sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de mousse, de stick, de dispersion de vésicules notamment de lipides ioniques ou non, de lotion biphase ou multiphase, de spray, de poudre, de pâte.

La composition peut être une composition anhydre, c'est-à-dire une composition contenant moins de 2 % en poids d'eau, voire moins de 0,5 % d'eau, notamment exempte d'eau, l'eau n'étant pas ajoutée lors de la préparation de la composition mais correspondant à l'eau résiduelle apportée par les ingrédients mélangés. La composition peut également se présenter sous forme de laque.

La composition décrite ci-dessus peut être mise en oeuvre sur cheveux secs ou humides. Les additifs décrits précédemment, lorsqu'ils sont présents, peuvent être appliqués sur les cheveux simultanément avec la composition de l'invention ou séparément. La composition peut être rincée ou non. Il est aussi possible d'effectuer ensuite un lavage des cheveux, ce lavage n'étant pas obligatoire.

On peut également utiliser un procédé d'application avec chauffage. Selon ce mode particulier, l'application sur les cheveux est mise en oeuvre par exemple au moyen d'un peigne, d'un pinceau à l'aide d'une brosse ou aux doigts.

L'application de la composition est ensuite suivie d'un séchage à une température supérieure à 40 °C. Selon un mode de réalisation particulier, cette température est supérieure à 45 °C. Selon un autre mode de réalisation particulier, cette température est supérieure à 45 °C et inférieure à 220 °C.

Le séchage peut être réalisé immédiatement après l'application ou après un temps de pose pouvant aller de 1 minute à 30 minutes.

De préférence, les cheveux sont séchés, en plus d'un apport de chaleur, avec un flux d'air. Ce flux d'air pendant le séchage permet d'améliorer l'individualisation du gainage.

Durant le séchage, une action mécanique sur les mèches peut être exercée telle qu'un peignage, un brossage, le passage des doigts.

L'étape de séchage du procédé de l'invention peut être mise en oeuvre avec un casque, un sèche-cheveux, un fer à lisser...

Lorsque l'étape de séchage est mise en oeuvre avec un casque ou un sèche-cheveux, la température du séchage est comprise entre 40 et 110 degrés, de préférence entre 50 et 90 degrés.

Lorsque l'étape de séchage est mise en oeuvre avec un fer à lisser, la température du séchage est comprise entre 110 et 220 degrés, de préférence entre 140 et 200 degrés.

Une fois le séchage terminé, un rinçage ou un shampooing terminal peut éventuellement être réalisé.

L'invention va être plus complètement illustrée à l'aide des exemples non limitatifs suivants.

### EXEMPLES

### Exemple 1 :

Les compositions suivantes sont préparées :

| Composition | A | A' (hors invention) |
|---|---|---|
| BioPSA DC 7-4405 à 40% dans l'isododécane commercialisé par Dow Corning | 17.5 g | 17.5 g |
| Gomme de PDMS alpha-omega dihydroxyle de très haut poids moléculaire | 1.5 g | 1.5 g |
| Mélange n-undécane : n-tridécane, la quantité de n-undécane étant majoritaire dans le mélange | Qs 100 g | - |
| Isododécane | - | Qs 100 g |

| | | |
|---|---|---|
| * tel que préparé selon la demande WO2008/155059 | | |

0,3 g de la composition A est appliqué sur une mèche de 1 g de cheveux de hauteur de ton 4 propres et humides. Après 2 minutes de pause, la mèche est brushée au sèche-cheveux à une température de 80°C pendant 2 minutes. On obtient une mèche dont les cheveux sont individualisés et corporisés, la corporisation obtenue est rémanente au shampooing.

Lorsque 0.3 g de la composition A' est appliqué dans les mêmes conditions sur une mèche de 1g de cheveux de hauteur de ton 4 propres et humides, le brushing de la mèche au sèche-cheveux est plus difficile à réaliser, la brosse passant moins facilement dans les cheveux.

### Exemple 2 :

La composition suivant est préparée :

| Composition | |
|---|---|
| BioPSA DC 7-4405 à 40% dans l'isododécane commercialisé par Dow Corning | 17.5 g |
| Gomme de PDMS alpha-omega dihydroxyle de très haut poids moléculaire | 1.5 g |
| Polyméthylsilsesquioxane commercialisé sous le nom Wacker Belsil PMS MK Powder par la société Wacker | 3 g |
| Disteardimonium hectorite (10%) et propylene carbonate (3%) dans l'isododécane commercialisé par Elementis sous le nom Bentone Gel ISD V | 15 g |
| Mélange n-undécane : n-tridécane dans lequel le n-undécane est majoritaire * | 30 g |
| Isododécane | Qs 100 g |

| | |
|---|---|
| * tel que préparé selon la demande WO2008/155059 | |

0,3 g de la composition est appliqué sur une mèche de 1g de cheveux de hauteur de ton 4 propres et humides. Après 2 minutes de pause, la mèche est séchée au sèche-cheveux à une température de 80°C pendant 2 minutes. On obtient une mèche dont les cheveux sont individualisés et corporisés, la corporisation obtenue est rémanente au shampooing.

### Exemple 3 :

Les compositions suivantes sont préparées :

| Composition | B | B' (hors invention) |
|---|---|---|
| BioPSA DC 7-4405 à 40% dans l'isododécane commercialisé par Dow Corning | 17.5 g | 17.5 g |
| Gomme de PDMS alpha-omega dihydroxyle de très haut poids moléculaire | 1.5 g | 1.5 g |
| Nacre mica enrobé d'oxyde de fer brun commercialisée par Eckart sous le nom Prestige Soft Bronze | 5 g | 5 g |
| Mélange n-undécane : n-tridécane dans lequel le n-undécanee est majoritaire* | Qs 100 g | - |
| Isododécane | - | Qs 100 g |

| | | |
|---|---|---|
| * tel que préparé selon la demande WO2008/155059 | | |

0,6 g de la composition B est appliqué sur une mèche de 1 g de cheveux de hauteur de ton 4 propres et humides. Après 2 minutes de pause, la mèche est brushée au sèche-cheveux à une température de 80°C pendant 2 minutes. On obtient une mèche colorée dont les cheveux sont individualisés et dont la couleur est très homogène et rémanente au shampooing.

Lorsque 0.6 g de la composition B' est appliqué dans les mêmes conditions sur une mèche de 1g de cheveux de hauteur de ton 4 propres et humides, le brushing de la mèche au sèche-cheveux est plus difficile à réaliser, la brosse passant moins facilement dans les cheveux.

### Exemple 4:

La composition suivante est réalisée :

| Composition | |
|---|---|
| BioPSA DC 7-4405 à 40% dans l'isododécane commercialisé par Dow Corning | 17.5 g |
| Gomme de PDMS alpha-omega dihydroxyle de haut poids moléculaire | 1.5 g |
| Nacre mica enrobé d'oxyde de fer brun commercialisée par Eckart sous le nom Prestige Soft Bronze | 5 g |
| Polyméthylsilsesquioxane commercialisé sous le nom Wacker Belsil PMS MK Powder par la société Wacker | 3 g |
| Disteardimonium hectorite (10%) et propylene carbonate (3%) dans l'isododécane commercialisé par Elementis sous le nom Bentone Gel ISD V | 15 g |
| Mélange n-undécane : n-tridécane dans lequel le n-undécane est majoritaire* | 20 g |
| Isododécane | Qs 100 g |

| | |
|---|---|
| * tel que préparé selon la demande WO2008/155059 | |

0,6 g de la composition est appliqué sur une mèche de 1g de cheveux de hauteur de ton 4 propres et humides. Après 2 minutes de pause, la mèche est séchée au sèche-cheveux à une température de 80°C pendant 2 minutes. On obtient une mèche colorée dont les cheveux sont individualisés et dont la couleur est très homogène et rémanente au shampooing.

### Exemple 5 :

La composition suivante est réalisée :

| Composition | |
|---|---|
| BioPSA DC 7-4405 à 40% dans l'isododécane commercialisé par Dow Corning | 17.5 g |
| Gomme de PDMS alpha-omega dihydroxyle de haut poids moléculaire | 1.5 g |
| Nacre mica enrobé d'oxyde de fer brun commercialisée par Eckart sous le nom Prestige Soft Bronze | 5 g |
| Polyméthylsilsesquioxane commercialisé sous le nom Wacker Belsil PMS MK Powder par la société Wacker | 3 g |
| Disteardimonium hectorite (10%) et propylene carbonate (3%) dans l'isododécane commercialisé par Elementis sous le nom Bentone Gel ISD V | 15 g |
| Mélange n-undécane : n-tridécane dans lequel le n-undécane étant majoritaire dans le mélange* | Qs 100 g |

| | |
|---|---|
| * tel que préparé selon la demande WO2008/155059 0,6 g de la composition est appliqué sur une mèche de 1g de cheveux de hauteur de ton 4 propres et humides. Après 2 minutes de pause, la mèche est séchée au sèche-cheveux à une température de 80°C pendant 2 minutes. On obtient une mèche colorée dont les cheveux sont individualisés et dont la couleur est très homogène et rémanente au shampooing. | |

## Revendications

1. Composition de traitement des fibres kératiniques comprenant un ou plusieurs copolymères à base de résine de silicone et de silicone fluide, au moins deux alcanes linéaires volatils liquides distincts, différant entre eux d'un nombre de carbone n d'au moins 1 les alcanes linéaires volatils liquides comprenant de 9 à 15 atomes de carbone, le rapport en poids entre les alcanes linéaires volatils liquides et le copolymère à base de résine de silicone et de silicone fluide étant supérieur ou égal à 2.

2. Composition selon la revendication 1 dans laquelle le copolymère comprend la résine de silicone à un taux compris entre 45 et 75 % et la silicone fluide à un taux compris entre 25 et 55 %, avec la somme des pourcentages de résine de silicone et de silicone fluide qui est égal à 100.

3. Composition selon la revendication 1 ou 2 dans laquelle la résine de silicone est présente à un taux compris entre 55 et 65 % et la silicone fluide est présente à un taux compris entre 35 et 45 %, avec la somme des pourcentages de résine de silicone et de silicone fluide qui est égal à 100.

4. Composition selon l'une quelconque des revendications précédentes dans laquelle la quantité de copolymère est supérieure à 1 % en poids du poids total de la composition.

5. Composition selon la revendication précédente dans laquelle l'alcane linéaire volatil comprend de 10 à 15 atomes de carbone, et plus particulièrement de 11 à 13 atomes de carbone.

6. Composition selon l'une quelconque des revendications précédentes dans laquelle l'alcane linéaire volatil est d'origine végétale.

7. Composition selon l'une quelconque des revendications précédentes, dans laquelle l'alcane linéaire volatil est choisi parmi le n-nonane, le n-undécane, le n-dodécane, le n-tridécane, le n-tétradécane, et leurs mélanges.

8. Composition selon l'une quelconque des revendications précédentes, comprenant au moins deux alcanes linéaires volatils distincts, différant entre eux d'un nombre de carbone de 1 ou de 2.

9. Composition selon l'une quelconque des revendications précédentes, comprenant un mélange d'au moins deux alcanes linéaires volatils comprenant
de 50 à 90% en poids du mélange, de préférence de 55 à 80% en poids, préférentiellement encore de 60 à 75% en poids, d'un alcane linéaire volatil en Cn, et
de 10 à 50% en poids du mélange, de préférence de 20 à 45% en poids, de préférence de 24 à 40% en poids, d'un alcane linéaire volatil en Cn+x avec x supérieur ou égal à 1 et de préférence x=1 ou x=2, par rapport au poids total des alcanes dans ledit mélange.

10. Composition selon la revendication précédente, comprenant un mélange n-undécane: n-tridécane (C11/C13) comprenant de 55 à 80% en poids, de préférence de 60 à 75% en poids d'alcane linéaire volatil en C11 (n-undécane), et de 20 à 45% en poids, de préférence de 24 à 40% en poids, d'alcane linéaire volatil en C13 (n-tridécane) par rapport au poids total des alcanes dans ledit mélange.

11. Composition selon la revendication 9 comprenant un mélange n-dodécane : n-tétradécane (C12/C14) comprenant de 65 à 95% en poids, de préférence de 70 à 90% en poids d'alcane linéaire volatil en C12 (n-dodécane) et de 5 à 35% en poids, de préférence de 10 à 30% en poids, d'alcane linéaire volatil en C14 (n-tétradécane) par rapport au poids total des alcanes dans ledit mélange.

12. Composition selon l'une quelconque des revendications précédentes, comprenant de 0,5 % à 90 % en poids, en particulier de 1 % à 90 % en poids, et plus particulièrement de 5 % à 90 % en poids d'alcanes linéaires volatils par rapport au poids total de la composition.

13. Composition selon l'une quelconque des revendications précédentes dans laquelle le rapport entre les alcanes linéaires volatils liquides et le copolymère à base de résine de silicone et de silicone fluide est compris entre 2 et 100 et de préférence entre 2.5 et 50.

14. Composition selon l'une quelconque des revendications précédentes comprenant un ou plusieurs composés siliconés additionnels choisis parmi les polysiloxanes présentant une viscosité supérieure à 100 cst, de préférence choisis parmi les huiles de silicone et les résines de silicone, encore plus particulièrement choisis parmi les huiles de type polydiméthylsiloxanes et les résines de silicones.

15. Composition selon l'une quelconque des revendications précédentes comprenant un ou plusieurs pigments, de préférence des nacres.

16. Composition selon l'une quelconque des revendications précédentes anhydre.

17. Procédé de traitement des fibres kératiniques comprenant l'application d'une composition telle que définie à l'une quelconque des revendications 1 à 15, cette application pouvant être suivie d'un séchage à une température supérieure à 40 °C.

## Patentansprüche

1. Zusammensetzung zur Behandlung von Keratinfasern, umfassend ein oder mehrere Copolymere auf Basis von Silikonharz und fließfähigem Silikon, mindestens zwei verschiedene flüssige flüchtige lineare Alkane, die sich voneinander durch eine Kohlenstoffzahl n von mindestens 1 unterscheiden, wobei die flüssigen flüchtigen linearen Alkane 9 bis 15 Kohlenstoffatome umfassen, wobei das Gewichtsverhältnis zwischen den flüssigen flüchtigen linearen Alkanen und dem Copolymer auf Basis von Silikonharz und fließfähigem Silikon größer gleich 2 ist.

2. Zusammensetzung nach Anspruch 1, wobei das Copolymer das Silikonharz in einem Gehalt zwischen 45 und 75% und das fließfähige Silikon in einem Gehalt zwischen 25 und 55% umfasst, wobei die Summe der Prozentanteile des Silikonharzes und des fließfähigen Silikons gleich 100 ist.

3. Zusammensetzung nach Anspruch 1 oder 2, wobei das Silikonharz in einem Gehalt zwischen 55 und 65% und das fließfähige Silikon in einem Gehalt zwischen 35 und 45% vorliegt, wobei die Summe der Prozentanteile des Silikonharzes und des fließfähigen Silikons gleich 100 ist.

4. Zusammensetzung nach einem der vorhergehenden Ansprüche, wobei die Copolymermenge über 1 Gew.-%, bezogen auf das Gesamtgewicht der Zusammensetzung, liegt.

5. Zusammensetzung nach dem vorhergehenden Anspruch, wobei das flüchtige lineare Alkan 10 bis 15 Kohlenstoffatome und spezieller 11 bis 13 Kohlenstoffatome umfasst.

6. Zusammensetzung nach einem der vorhergehenden Ansprüche, wobei das flüchtige lineare Alkan pflanzlicher Herkunft ist.

7. Zusammensetzung nach einem der vorhergehenden Ansprüche, wobei das flüchtige lineare Alkan aus n-Nonan, n-Undecan, n-Dodecan, n-Tridecan, n-Tetradecan und Mischungen davon ausgewählt ist.

8. Zusammensetzung nach einem der vorhergehenden Ansprüche, umfassend mindestens zwei verschiedene flüchtige lineare Alkane, die sich voneinander durch eine Kohlenstoffzahl von 1 oder 2 unterscheiden.

9. Zusammensetzung nach einem der vorhergehenden Ansprüche, umfassend eine Mischung von mindestens zwei flüchtigen linearen Alkanen, umfassend 50 bis 90 Gew.-% der Mischung, vorzugsweise 55 bis 80 Gew.-%, weiter bevorzugt 60 bis 75 Gew.-%, eines flüchtigen linearen Cn-Alkans und 10 bis 50 Gew.-% der Mischung, vorzugsweise 20 bis 45 Gew.-%, weiter bevorzugt 24 bis 40 Gew.-%, eines flüchtigen linearen Cn+x-Alkans, wobei x größer gleich 1 und vorzugsweise x=1 oder x=2 ist, bezogen auf das Gesamtgewicht der Alkane in der Mischung.

10. Zusammensetzung nach dem vorhergehenden Anspruch, umfassend eine n-Undecan:n-Tridecan-(C11/C13) - Mischung, umfassend 55 bis 80 Gew.-%, vorzugsweise 60 bis 75 Gew.-%, flüchtiges lineares C11-Alkan (n-Undecan) und 20 bis 45 Gew.-%, vorzugsweise 24 bis 40 Gew.-%, flüchtiges lineares C13-Alkan (n-Tridecan), bezogen auf das Gesamtgewicht der Alkane in der Mischung.

11. Zusammensetzung nach Anspruch 9, umfassend eine n-Dodecan:n-Tetradecan-(C12/C14)-Mischung, umfassend 65 bis 95 Gew.-%, vorzugsweise 70 bis 90 Gew.-%, flüchtiges lineares C12-Alkan (n-Dodecan) und 5 bis 35 Gew.-%, vorzugsweise 10 bis 30 Gew.-%, flüchtiges lineares C14-Alkan (n-Tetradecan), bezogen auf das Gesamtgewicht der Alkane in der Mischung.

12. Zusammensetzung nach einem der vorhergehenden Ansprüche, umfassend 0,5 bis 90 Gew.-%, insbesondere 1 bis 90 Gew.-% und spezieller 5 bis 90 Gew.-% flüchtige lineare Alkane, bezogen auf das Gesamtgewicht der Zusammensetzung.

13. Zusammensetzung nach einem der vorhergehenden Ansprüche, wobei das Verhältnis zwischen den flüssigen flüchtigen linearen Alkanen und dem Copolymer auf Basis von Silikonharz und fließfähigem Silikon zwischen 2 und 100 und vorzugsweise zwischen 2,5 und 50 liegt.

14. Zusammensetzung nach einem der vorhergehenden Ansprüche, umfassend eine oder mehrere zusätzliche Silikonverbindungen, ausgewählt aus Polysiloxanen mit einer Viskosität von mehr als 100 cSt, vorzugsweise ausgewählt aus Silikonölen und Silikonharzen, noch spezieller ausgewählt aus Ölen vom Polydimethylsiloxan-Typ und Silikonharzen.

15. Zusammensetzung nach einem der vorhergehenden Ansprüche, umfassend ein oder mehrere Pigmente, vorzugsweise Perlmutt.

16. Zusammensetzung nach einem der vorhergehenden Ansprüche in wasserfreier Form.

17. Verfahren zur Behandlung von Keratinfasern, bei dem man eine Zusammensetzung gemäß einem der Ansprüche 1 bis 15 aufbringt und danach gegebenenfalls bei einer Temperatur von mehr als 40°C trocknet.

## Claims

1. Composition for treating keratin fibres, comprising one or more copolymers based on silicone resin and fluid silicone, and at least two different liquid volatile linear alkanes which differ from one another by a carbon number n of at least 1, the liquid volatile linear alkanes comprising from 9 to 15 carbon atoms, and the weight ratio between the liquid volatile linear alkanes and the copolymer based on silicone resin and fluid silicone being greater than or equal to 2.

2. Composition according to Claim 1, in which the copolymer comprises the silicone resin in a proportion of between 45% and 75% and the fluid silicone in a proportion of between 25% and 55%, with the sum of the percentages of silicone resin and fluid silicone being equal to 100.

3. Composition according to Claim 1 or 2, in which the silicone resin is present in a proportion of between 55% and 65% and the fluid silicone is present in a proportion of between 35% and 45%, with the sum of the percentages of silicone resin and fluid silicone being equal to 100.

4. Composition according to any of the preceding claims, in which the amount of copolymer is greater than 1% by weight of the total weight of the composition.

5. Composition according to the preceding claim, in which the volatile linear alkane comprises from 10 to 15 carbon atoms, and more particularly from 11 to 13 carbon atoms.

6. Composition according to any of the preceding claims, in which the volatile linear alkane is of plant origin.

7. Composition according to any of the preceding claims, in which the volatile linear alkane is selected from n-nonane, n-undecane, n-dodecane, n-tridecane, n-tetradecane, and mixtures thereof.

8. Composition according to any of the preceding claims, comprising at least two different volatile linear alkanes which differ from one another by a carbon number of 1 or of 2.

9. Composition according to any of the preceding claims, comprising a mixture of at least two volatile linear alkanes, comprising
from 50% to 90% by weight of the mixture, preferably from 55% to 80% by weight, more preferably from 60% to 75% by weight, of a Cn volatile linear alkane, and
from 10% to 50% by weight of the mixture, preferably from 20% to 45% by weight, preferably from 24% to 40% by weight, of a Cn + x volatile linear alkane, with x being greater than or equal to 1, and preferably x = 1 or x = 2, relative to the total weight of the alkanes in said mixture.

10. Composition according to the preceding claim, comprising an n-undecane/n-tridecane (C11/C13) mixture, comprising from 55% to 80% by weight, preferably from 60% to 75% by weight, of C11 volatile linear alkane (n-undecane), and from 20% to 45% by weight, preferably from 24% to 40% by weight, of C13 volatile linear alkane (n-tridecane), relative to the total weight of the alkanes in said mixture.

11. Composition according to Claim 9, comprising an n-dodecane/n-tetradecane (C12/C14) mixture, comprising from 65% to 95% by weight, preferably from 70% to 90% by weight, of C12 volatile linear alkane (n-dodecane), and from 5% to 35% by weight, preferably from 10% to 30% by weight, of C14 volatile linear alkane (n-tetradecane), relative to the total weight of the alkanes in said mixture.

12. Composition according to any of the preceding claims, comprising from 0.5% to 90% by weight, in particular from 1% to 90% by weight, and more particularly from 5% to 90% by weight of volatile linear alkanes, relative to the total weight of the composition.

13. Composition according to any of the preceding claims, in which the ratio between the liquid volatile linear alkanes and the copolymer based on silicone resin and fluid silicone is between 2 and 100 and preferably between 2.5 and 50.

14. Composition according to any of the preceding claims, comprising one or more additional silicone compounds selected from polysiloxanes having a viscosity of more than 100 cSt, preferably selected from silicone oils and silicone resins, more particularly selected from polydimethylsiloxane oils and silicone resins.

15. Composition according to any of the preceding claims, comprising one or more pigments, preferably nacres.

16. Composition according to any of the preceding claims, in anhydrous form.

17. Method of treating keratin fibres, comprising applying a composition as defined in any of Claims 1 to 15, it being possible for said applying to be followed by drying at a temperature greater than 40°C.
